# EUROPÄISCHE PATENTSCHRIFT

(11) **EP 3 415 903 B1**
(45) Veröffentlichungstag und Bekanntmachung des Hinweises auf die Patenterteilung: **04.05.2022**
(21) Anmeldenummer: 18167763.4
(22) Anmeldetag: 17.04.2018
(51) Int. Cl.: G01N 33/00

(54) **VERFAHREN ZUR KALIBRIERUNG EINES GASSENSORS**
METHOD FOR CALIBRATION OF A GAS SENSOR
PROCÉDÉ D'ÉTALONNAGE D'UN CAPTEUR DE GAZ

(30) Priorität: 17.05.2017 DE 102017004727
(43) Veröffentlichungstag der Anmeldung: 19.12.2018
(73) Patentinhaber: Drägerwerk AG & Co. KGaA, 23558 Lübeck (DE)
(72) Erfinder: Martens, Matthias, 23860 Groß Schenkenberg (DE); Hansmann, Hans-Ullrich, 23858 Barnitz (DE); Einecke, Kai, 23919 Berkenthin (DE); Buchner, Rainer, 23909 Ratzeburg (DE); Sturm, Hannes, 22926 Ahrensburg (DE)

(56) Entgegenhaltungen:
- DE-A1-102010 015 994
- DE-T2- 60 102 837
- DE-U1- 29 521 224
- US-A- 4 384 925
- US-B1- 6 632 674

## Beschreibung

Die Erfindung betrifft ein Verfahren zum Betrieb eines Gassensors, nämlich ein Verfahren zur Kalibrierung des Gassensors und zur anschließenden Verwendung des kalibrierten Gassensors.

Ein Verfahren zur Kalibrierung eines Gassensors ist aus der US 2011/0197649 A1 bekannt. Dort ist vorgesehen, dass mittels mehrerer Ventile ein Volumen abgeschlossen werden kann, in dem ein zu messendes Gas mittels einer Pumpe durch einen Gassensor zirkuliert wird. Das abgeschlossene Volumen gewährleistet bekannte Verhältnisse als Basis für eine Konzentrationsberechnung.

In der WO 2007/087403 A1, der US 6,632,674 und der US 4,384,925 sind Verfahren zur Kalibrierung eines Gassensors beschrieben, die - kurz gefasst - auf einer Freisetzung einer bekannten Stoffmenge basieren. Bei der US 4,384,925 wird ein Prüfgas mit einer bekannten (kalibrierten) Menge des zu sensierenden Stoffs freigesetzt, wobei die bekannte Menge gespeichert ist und der bekannte Wert und ein in Bezug auf das Prüfgas sensierter Wert zur Kalibrierung verwendet werden.

Bei bekannten, sogenannten blendengesteuerten Gassensoren ist deren Messsignal von einem Stofftransport durch eine Membran des Gassensors abhängig. Die Membran und deren Dichtungselemente trennen die eigentliche Sensorik und einen Elektrolyten im Innern des Sensors von einem jeweiligen Messgas und verhindern bis zu einer konstruktiv gegebenen Druckdifferenz ein Austreten des Elektrolyten.

Typische Kalibrierungsverfahren geben eine bestimmte Prüfgaskonzentration vor und prüfen, ob der Sensor einen in weiten Grenzen richtigen Messwert berechnet. Dabei wird eine Differenz zwischen einem aufgrund der bekannten Prüfgaskonzentration resultierenden Erwartungswert und dem jeweiligen Messwert zur Korrektur verwendet. Idealerweise liegt die Prüfgaskonzentration in einem beim späteren Betrieb des Gassensors zu überwachenden Bereich oder unterliegt normativen Anforderungen.

Ein wechselnder Druck auf die Trennmembran des Gassensors führt zu einem druckabhängigen Stoffdurchgang durch die Membran (erhöhter Stoffdurchgang bei hohem Druck; geringerer Stoffdurchgang bei geringem Druck).

Deshalb zeigen diejenigen Gassensoren, deren Funktionsprinzip auf einem konzentrationsabhängigen Stoffdurchgang beruht, bei einem hohen Druck erhöhte Messwerte. Druckwechsel, welche die Messwerte eines Gassensors merklich beeinflussen (verfälschen), werden häufig durch eine Pumpe verursacht, welche das zu messende Gas über eine längere Zuleitung (zum Beispiel Schlauch oder Rohr) zu dem Gassensor transportiert. Eine solche Pumpe umfasst häufig ein rotatorisch betriebenes elastisches Kammervolumen oder eine Kolben- / Zylindereinheit, welche beide zyklisch arbeiten und folglich zyklische Druckschwankungen verursachen.

Neben dem Druckwechsel, der zu einem verfälschten Messwert führt, ist ein Wechsel der Gaskonzentration eine weitere Fehlerquelle. Die Kalibrierung eines Gassensors ist auf ein ruhendes oder im Wesentlichen ruhendes Medium in einem Bereich (Gasraum) vor der Membran abgestimmt. Bei einem ruhenden Medium entsteht in unmittelbarer Nähe zu der Membran eine sogenannte Verarmungsschicht mit einer geringeren Konzentration des zu messenden Gases. Die zu messenden Moleküle, die durch das Konzentrationsgefälle durch die Membran hindurchtreten, fehlen auf der Gegenseite der Membran und werden kurzfristig durch Diffusion aus dem Gasraum ausgeglichen. Direkt vor der Membran befindet sich eine geringere Konzentration als in dem zu messenden Gas. Wird nun in den Raum direkt vor der Membran Energie eingebracht, so wird diese Konzentrationsschichtung verändert. Der Energieeintrag ergibt sich beispielsweise aufgrund einer durch einen (variierenden) Volumenstrom verursachten Gasbewegung.

Die jeweils wirksamen Druck- und/oder Volumenstromverhältnisse beeinflussen also das Messergebnis. Zum einen wird ein fehlerhafter Messwert angezeigt. Zum anderen wird eine Konzentrationsänderung angezeigt, obwohl die Gaskonzentration gleich geblieben ist.

Eine Aufgabe der vorliegenden Erfindung besteht entsprechend darin, ein Verfahren anzugeben, mit dem solche Fehler minimiert oder zumindest reduziert werden können.

Erfindungsgemäß wird diese Aufgabe mittels eines Verfahrens zum Betrieb eines Gassensors mit den Merkmalen des Anspruchs 1 sowie mittels einer nach dem Verfahren arbeitenden Vorrichtung gelöst. Das Verfahren umfasst eine Kalibrierung des Gassensors (Kalibrierungsverfahren) sowie eine Verwendung der Kalibrierung, also eine Verwendung des kalibrierten Gassensors (Messverfahren).

Bei dem hier vorgeschlagenen Kalibrierungsverfahren, also einem zeitlich ersten Teil des Verfahrens zum Betrieb eines Gassensors, ist vorgesehen, dass mittels einer mit dem Gassensor fluidisch gekoppelten Pumpe ein Prüfgas eingesaugt und zum Gassensor gefördert wird, dass mittels der Pumpe unterschiedliche Volumenströme erzeugt werden und mittels des Gassensors jeweils volumenstromabhängige Messwerte (Gasmesswerte) aufgenommen werden und dass die volumenstromabhängigen Messwerte zusammen mit dem jeweiligen Volumenstrom, also einem den jeweiligen Volumenstrom kodierenden Wert (Volumenstromwert), zum Beispiel einem sensorisch erfassten Volumenstrommesswert oder einem für die Ansteuerung der Pumpe verwendeten Sollwert, für eine spätere Verwendung aufgezeichnet werden.

Die im Rahmen des Kalibrierungsverfahrens aufgenommenen Daten, nämlich Paare von Gasmesswerten und Volumenstromwerten, bilden die Basis für die Kalibrierung eines später beim Messbetrieb mittels des Gassensors aufgenommenen Gasmesswerts. Die im Rahmen des Kalibrierungsverfahrens aufgenommenen Daten sowie daraus optional resultierende Daten werden im Folgenden zusammenfassend kurz als Kalibrierungsbasis bezeichnet.

Bei einem an das Kalibrierungsverfahren anschließenden, also zeitlich späteren zweiten Teil des Verfahrens, nämlich einem Messbetrieb des Gassensors, werden einerseits ein von dem Gassensor gelieferter Messwert und ein wirksamer Volumenstrom erfasst. Die Erfassung des Messwerts (Gasmesswert) mittels des Gassensors sowie die Aufnahme eines den jeweiligen Volumenstrom kodierenden Werts erfolgt genauso wie während des Kalibrierungsverfahrens. Der während des Messbetriebs erfasste Gasmesswert wird anhand der Daten der Kalibrierungsbasis korrigiert. Dabei wird der während des Messbetriebs erfasste Gasmesswert anhand des erfassten, während der Aufnahme dieses Gasmesswerts wirksamen Volumenstroms sowie anhand der im Rahmen des Kalibrierungsverfahrens ermittelten volumenstromabhängigen Gasmesswerte korrigiert.

Im Rahmen des hier vorgeschlagenen Verfahrens zum Betrieb eines Gassensors werden also Gasmesswerte in unterschiedlichen Situationen, nämlich einerseits während des Kalibrierungsverfahrens und andererseits während des späteren Messbetriebs, aufgenommen und jeweils zugehörige Volumenstromwerte, die einen während der Aufnahme des jeweiligen Gasmesswerts wirksamen Volumenstrom kodieren, aufgenommen. Zur Unterscheidung werden die Gasmesswerte mitunter als Kalibrierungs-Gasmesswerte bezeichnet, wenn deren Aufnahme im Rahmen des Kalibrierungsverfahrens erfolgt, und als Betriebs-Gasmesswerte bezeichnet, wenn deren Aufnahme während des Messbetriebs erfolgt. Gleiches gilt für die Volumenstromwerte, die mitunter entsprechend als Kalibrierungs-Volumenstromwerte bzw. Betriebs-Volumenstromwerte bezeichnet werden.

Unter Verwendung dieser Terminologie kann das hier vorgeschlagene Verfahren wie folgt beschrieben werden: Zur Kalibrierung des Gassensors wird mittels einer mit dem Gassensor gekoppelten Pumpe ein Prüfgas eingesaugt und zum Gassensor gefördert. Mittels der Pumpe werden unterschiedliche Volumenströme erzeugt und Kalibrierungs-Volumenstromwerte aufgenommen. Mittels des Gassensors werden während der Wirkung unterschiedlicher Volumenströme jeweils volumenstromabhängige Kalibrierungs-Gasmesswerte aufgenommen. Auf Basis der volumenstromabhängigen Kalibrierungs-Gasmesswerte und der zugehörigen Kalibrierungs-Volumenstromwerte werden als Kalibrierungsbasis zum Beispiel Korrekturfaktoren oder eine Kalibrierfunktion ermittelt.

Beim anschließenden Messbetrieb des Gassensors werden die Daten der Kalibrierungsbasis verwendet. Dabei werden ein vom Gassensor gelieferter Betriebs-Gasmesswert, also ein aktueller Messwert, und ein während der Erfassung des Betriebs-Gasmesswerts wirksamer Betriebs-Volumenstromwert erfasst. Der Betriebs-Gasmesswert wird anhand des Betriebs-Volumenstromwerts sowie anhand der im Rahmen des Kalibrierungsverfahrens ermittelten volumenstromabhängigen Kalibrierungs-Gasmesswerte korrigiert. Diese Korrektur erfolgt beispielsweise, indem die Kalibrierfunktion oder die Korrekturfaktoren zur Kalibrierung des erfassten Betriebs-Gasmesswerts verwendet werden, nämlich zum Beispiel indem abhängig von dem erfassten Betriebs-Volumenstromwert ein zu diesem am besten passender Korrekturfaktor oder ein zu diesem gehöriger Wert der Kalibrierfunktion ermittelt und der Betriebs-Gasmesswert damit kalibriert wird.

Im Interesse einer besseren Lesbarkeit der nachfolgenden Beschreibung wird diese mit den kürzeren Begriffen "Messwert" und "Volumenstromwert" fortgesetzt. Je nach jeweiligem Sachzusammenhang sind die Begriff als Kalibrierungs-Gasmesswert oder Betriebs-Gasmesswert sowie Kalibrierungs-Volumenstromwert oder Betriebs-Volumenstromwert zu lesen.

Es sei darauf hingewiesen, dass der Messbetrieb nicht notwendig unmittelbar an das Kalibrierungsverfahren anschließt. Tatsächlich kommt es auf einen zeitlichen Abstand zwischen dem Kalibrierungsverfahren und dem Messbetrieb nicht an. Das Kalibrierungsverfahren kann ggf. öfter, zum Beispiel regelmäßig zu vorgegebenen oder vorgebbaren Zeitpunkten ausgeführt werden, wenn der Abstand zwischen einer Aufnahme eines Messwerts im Messbetrieb und dem Kalibrierungsverfahren zu groß ist oder auch nur Anlass zu der Annahme besteht, dass der zeitliche Abstand zur Kalibrierung zu groß ist.

Der Vorteil des vorgeschlagenen Kalibrierungsverfahrens besteht darin, dass durch die Aufzeichnung der volumenstromabhängigen Messwerte (volumenstromabhängige Kalibrierungs-Gasmesswerte) zusammen mit dem jeweiligen Volumenstrom (Kalibrierungs-Volumenstromwerte) die tatsächliche Abhängigkeit der von dem Gassensor gelieferten Messwerte von dem jeweiligen Volumenstrom erfasst wird. Indem die volumenstromabhängigen Messwerte zusammen mit dem jeweiligen Volumenstrom erfasst werden, kann beim späteren Messbetrieb anhand eines dann jeweils wirksamen Volumenstroms auf die volumenstromabhängigen Messwerte oder eine jeweilige Repräsentation der volumenstromabhängigen Messwerte, zum Beispiel eine Lookup-Tabelle oder ein Polynom, zugegriffen werden.

Das Kalibrierungsverfahren wird bevorzugt ausgeführt, wenn der jeweilige Gassensor und die diesem zugeordnete Pumpe sich in einer realen Einbausituation befinden, denn neben der jeweiligen Pumpleistung der Pumpe hat auch ein Schlauch- oder Rohrleitungsabschnitt, mit dem Pumpe und Gassensor fluidisch gekoppelt sind, sowie ein Schlauch- oder Rohrleitungsabschnitt, mit dem das Prüfgas angesaugt wird, und ein Schlauch- oder Rohrleitungsabschnitt, durch den das Prüfgas ausgestoßen wird, Einfluss auf den jeweils beim Gassensor wirksamen Volumenstrom. Eine Kalibrierung unter den Bedingungen der jeweiligen Einbausituation liefert folglich volumenstromabhängige Messwerte, mit denen eine besonders genaue Kalibrierung möglich ist.

Bei einem Verfahren zur Verwendung eines nach dem hier vorgeschlagenen Ansatz kalibrierten Gassensors werden beim Betrieb des Gassensors ein Messwert (originärer Messwert; Betriebs-Gasmesswert) und ein wirksamer Volumenstrom (Betriebs-Volumenstrom) erfasst. Der erfasste originäre Messwert wird sodann anhand des erfassten wirksamen Volumenstroms und der im Rahmen des Kalibrierungsverfahrens ermittelten volumenstromabhängigen Messwerte korrigiert. Der Gassensor gibt anschließend den korrigierten Messwert als Messwert für die sensierte Gaskonzentration aus.

Bei einer Ausführungsform eines solchen Verfahrens zur Verwendung eines nach dem hier vorgeschlagenen Ansatz kalibrierten Gassensors wird anhand des erfassten Volumenstroms (Betriebs-Volumenstromwert) eine auf Basis der im Rahmen des Kalibrierungsverfahrens ermittelten volumenstromabhängigen Messwerte ermittelte Kalibrierungsbasis, zum Beispiel eine Kalibrierfunktion, ausgewählt, insbesondere wird anhand des erfassten Volumenstroms eine von mehreren Kalibrierungsbasen, zum Beispiel eine von mehreren Kalibrierfunktionen, ausgewählt, nämlich diejenige Kalibrierungsbasis oder Kalibrierfunktion, die zu dem erfassten Volumenstrom (Betriebs-Volumenstromwert) am besten passt. Der erfasste originäre Messwert (Betriebs-Gasmesswert) wird sodann anhand der ausgewählten Kalibrierungsbasis oder Kalibrierfunktion korrigiert. Der Gassensor gibt anschließend den korrigierten Messwert als Messwert für die sensierte Gaskonzentration aus.

Das Kalibrierungsverfahren und die anschließende Korrektur eines beim Messbetrieb des Gassensors aufgenommenen Messwerts (Betriebs-Gasmesswert) sowie nachfolgend beschriebene Ausführungsformen des Kalibrierungsverfahrens sowie der Korrektur des Messwerts und die davon umfassten Verfahrensschritte werden automatisch ausgeführt, also ohne einen besonderen Eingriff eines Benutzers des Gassensors. Die automatische Ausführung der Verfahrensschritte erfolgt unter Kontrolle einer Steuerungseinheit, insbesondere einer dem Gassensor oder der Pumpe zugeordneten Steuerungseinheit. Diese umfasst eine Verarbeitungseinheit in Form von oder nach Art eines Mikroprozessors sowie einen Speicher. In den Speicher ist ein von der Verarbeitungseinheit ausführbares Steuerungsprogramm geladen oder ladbar, das beim Betrieb durch dessen Verarbeitungseinheit ausgeführt wird. Bedienhandlungen des Benutzers im Zusammenhang mit dem Verfahren beschränken sich zum Beispiel auf das Starten des Kalibrierungsverfahrens und/oder das Bereitstellen des Prüfgases.

Entsprechend wird die oben genannte Aufgabe auch mittels einer Steuerungseinheit gelöst, die nach dem Verfahren wie hier und im Folgenden beschrieben arbeitet und dazu Mittel zur Durchführung des Verfahrens umfasst. Die Erfindung ist dabei bevorzugt in Software implementiert.

Die Erfindung ist damit einerseits auch ein als Steuerungsprogramm fungierendes Computerprogramm mit durch einen Computer ausführbaren Programmcodeanweisungen und andererseits ein Speichermedium mit einem derartigen Computerprogramm, also ein Computerprogrammprodukt mit Programmcodemitteln, sowie schließlich auch eine Vorrichtung in Form eines Systems mit einem Gassensor und einer fluidisch mit dem Gassensor gekoppelten Pumpe sowie einer Steuerungseinheit, in deren Speicher als Mittel zur Durchführung des Verfahrens und seiner Ausgestaltungen ein solches Computerprogramm geladen oder ladbar ist.

Vorteilhafte Ausgestaltungen der Erfindung sind Gegenstand der Unteransprüche. Dabei verwendete Rückbeziehungen weisen auf die weitere Ausbildung des Gegenstandes des Hauptanspruches durch die Merkmale des jeweiligen Unteranspruches hin und sind nicht als ein Verzicht auf die Erzielung eines selbständigen, gegenständlichen Schutzes für die Merkmalskombinationen der rückbezogenen Unteransprüche zu verstehen. Des Weiteren ist im Hinblick auf eine Auslegung der Ansprüche sowie der Beschreibung bei einer näheren Konkretisierung eines Merkmals in einem nachgeordneten Anspruch davon auszugehen, dass eine derartige Beschränkung in den jeweils vorangehenden Ansprüchen sowie einer allgemeineren Ausführungsform des gegenständlichen Verfahrens nicht vorhanden ist. Jede Bezugnahme in der Beschreibung auf Aspekte nachgeordneter Ansprüche ist demnach auch ohne speziellen Hinweis ausdrücklich als Beschreibung optionaler Merkmale zu lesen.

Bei einer Ausführungsform des Kalibrierungsverfahrens ist vorgesehen, dass zur Erzeugung unterschiedlicher Volumenströme mittels der Pumpe anfänglich ein Volumenstrom entsprechend einem vorgegebenen Startwert erzeugt wird und dass ausgehend von dem Startwert der Volumenstrom erhöht oder verringert wird, bis ein vorgegebener Zielwert erreicht ist. Dies schafft im Vergleich zu der allgemeineren Form des Verfahrens, welches im Grunde lediglich vorsieht, dass mittels der Pumpe zum Beispiel zufällig oder quasi-zufällig unterschiedliche Volumenströme erzeugt werden, definierte und leicht reproduzierbare Verhältnisse.

Bei einer weiteren Ausführungsform des Kalibrierungsverfahrens ist vorgesehen, dass der Volumenstrom ausgehend vom dem Startwert mit einer vorgegebenen oder vorgebbaren Schrittweite schrittweise erhöht oder verringert wird, bis der Zielwert erreicht ist. Eine solche schrittweise (inkrementelle) Erhöhung oder Verringerung des Volumenstroms reduziert die im Rahmen des Kalibrierungsverfahrens anfallende Datenmenge. Für einen Volumenstrom entsprechend dem Start- und Zielwert werden jeweils ein volumenstromabhängiger Messwert (Kalibrierungs-Gasmesswert) und der zugehörige Volumenstrom (Kalibrierungs-Volumenstromwert) aufgenommen. Dazwischen werden nach jeder schrittweisen Erhöhung oder Verringerung des Volumenstroms nochmals jeweils ein volumenstromabhängiger Messwert (Kalibrierungs-Gasmesswert) und der zugehörige Volumenstrom (Kalibrierungs-Volumenstromwert) aufgenommen. Es ergibt sich dann zwar kein kontinuierlicher, die Abhängigkeit der vom Gassensor jeweils gelieferten Messwerte vom Volumenstrom beschreibender Verlauf, sondern ein diskreter Verlauf. Dies reicht aber als Basis für eine Interpolation von Zwischenwerten aus. Wenn eine besonders hohe Genauigkeit gefordert ist, kann die Schrittweite entsprechend angepasst (reduziert) werden und bei einer reduzierten Schrittweite werden entsprechend mehr volumenstromabhängige Messwerte und ein jeweils zugehöriger Volumenstrom aufgenommen.

Bei einer besonderen Ausführungsform des Kalibrierungsverfahrens ist der Startwert für den Volumenstrom größer als der Zielwert und entsprechend wird der Volumenstrom ausgehend von dem Startwert verringert, insbesondere mit einer vorgegebenen oder vorgebbaren Schrittweite schrittweise verringert, bis der Zielwert erreicht ist. Dann stellt sich aufgrund des im Zuge des Verfahrens bewirkten Spülens des Totraums in dem Schlauch- oder Rohrleitungsabschnitt mit dem anfänglich größeren Volumenstrom die entsprechende Gaskonzentration am Sensor schneller ein.

Bei einer weiteren Ausführungsform des Kalibrierungsverfahrens wird auf Basis der volumenstromabhängigen Messwerte (Kalibrierungs-Gasmesswert) und des jeweils zugehörigen Volumenstroms (Kalibrierungs-Volumenstromwert) automatisch eine Kalibrierfunktion ermittelt, zum Beispiel durch eine grundsätzlich an sich bekannte Polynominterpolation. Mittels der Kalibrierfunktion kann beim späteren Messbetrieb für jeden sich dann ergebenden Volumenstrom (Betriebs-Volumenstromwert) durch Einsetzen des Volumenstroms in die Kalibrierfunktion unmittelbar ein Kalibrierfaktor zur Korrektur des vom Gassensor originär gelieferten Messwerts (Betriebs-Gasmesswert) ermittelt werden.

Entsprechend ist bei einem Verfahren zur Verwendung eines mittels eines Kalibrierungsverfahren der hier und im Folgenden beschrieben Art kalibrierten Gassensors vorgesehen,
dass beim Betrieb des Gassensors ein von dem Gassensor originär gelieferter Messwert (Betriebs-Gasmesswert) und ein jeweils wirksamer Volumenstrom (Betriebs-Volumenstromwert) erfasst werden und
dass der erfasste Messwert (Betriebs-Gasmesswert) anhand des erfassten wirksamen Volumenstroms (Betriebs-Volumenstromwert) und der im Rahmen des Kalibrierungsverfahrens ermittelten volumenstromabhängigen Messwerte (Kalibrierungs-Volumenstromwerte) korrigiert wird.

Bei einer besonderen Ausführungsform dieses Verfahrens zur Verwendung eines nach dem hier beschriebenen Ansatz kalibrierten Gassensors erfolgt die Korrektur des von dem Gassensor originär gelieferten Messwerts anhand einer im Rahmen des Kalibrierungsverfahrens ermittelten Kalibrierfunktion.

Nachfolgend wird ein Ausführungsbeispiel der Erfindung anhand der Zeichnung näher erläutert. Einander entsprechende Gegenstände oder Elemente sind in allen Figuren mit den gleichen Bezugszeichen versehen.

Das oder jedes Ausführungsbeispiel ist nicht als Einschränkung der Erfindung zu verstehen.

Es zeigen
- Figur 1: einen Gassensor und eine Pumpe, wobei mittels der Pumpe dem Gassensor ein Prüfgas oder ein zu messendes Gas zugeführt wird,
- Figur 2: eine fiktive Abhängigkeit eines Messsignals des Gassensors von einem Volumenstrom durch den Gassensor,
- Figur 3: einen Messwerteverlauf beim Ablauf des hier vorgeschlagenen Kalibrierungsverfahrens,
- Figur 4: eine aufgrund des Kalibrierungsverfahrens resultierende Kalibrierungsfunktion,
- Figur 5: eine Darstellung des Kalibrierungsverfahrens in Form eines Flussdiagramms,
- Figur 6: eine Steuerungseinheit mit einem in einen Speicher der Steuerungseinheit geladenen Steuerungsprogramm als Mittel zur Ausführung des Kalibrierungsverfahrens,
- Figur 7: ein System mit einem Gassensor und einer Pumpe wie in Fig. 1 sowie einem eingangsseitigen Filter als Beispiel für einen in dem System wirkenden Strömungswiderstand und
- Figur 8: aufgrund unterschiedlicher Strömungswiderstände resultierende Kennlinien.

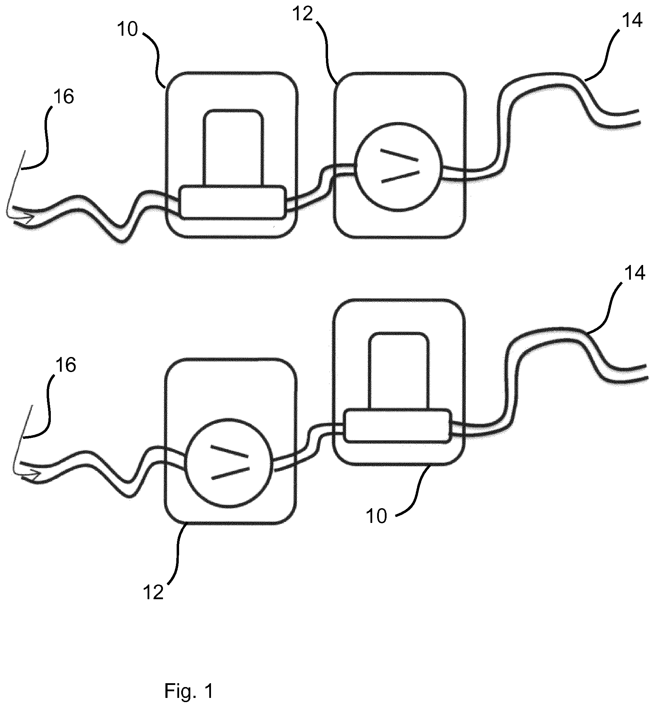

Die Darstellung in Figur 1 zeigt schematisch stark vereinfacht ein System mit einem Gassensor 10 und einer Pumpe 12 in zwei unterschiedlichen Konfigurationen. Der Gassensor 10 und die Pumpe 12 sind in einem Schlauchsystem 14 zusammengeschlossen (fluidisch gekoppelt).

Durch das Schlauchsystem 14 wird eingangsseitig ein Mess- oder Prüfgas 16 angesaugt und aufgrund der entweder stromaufwärts oder stromabwärts des Gassensors 10 befindlichen Pumpe 12 zum Gassensor 10 transportiert.

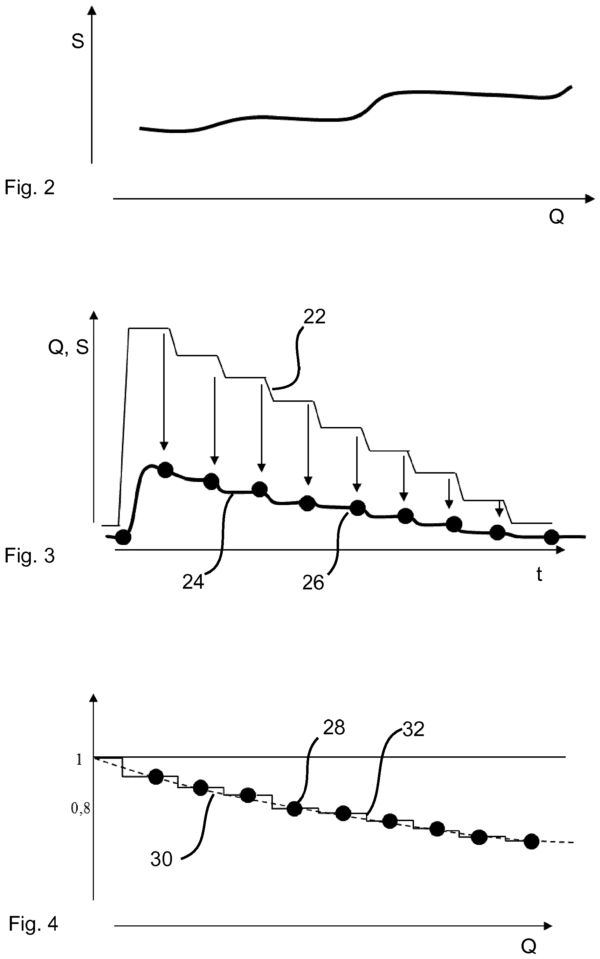

Die Darstellung in Figur 2 zeigt eine fiktive Abhängigkeit eines Messsignals S des Gassensors 10 von einem Volumenstrom Q durch den Gassensor 10, nämlich dem mittels der Pumpe 12 erzeugten Volumenstrom Q. In der Darstellung in Figur 2 ist der Volumenstrom Q auf der Abszisse und das Messsignal S des Gassensors 10 auf der Ordinate abgetragen.

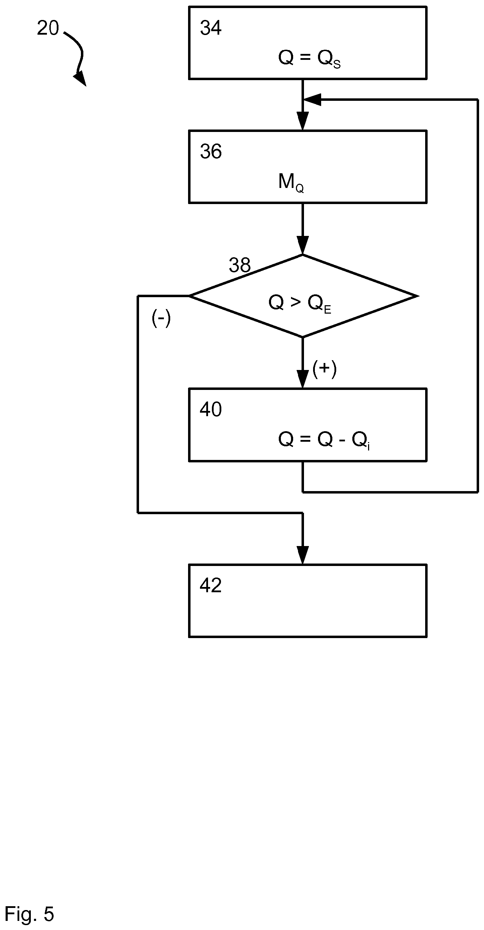

Die Darstellung in Figur 3 zeigt über der Zeit t den Ablauf eines hier vorgeschlagenen Kalibrierungsverfahrens 20 (Fig. 5). Die beiden Graphen zeigen einen Verlauf des Volumenstroms Q (Volumenstromverlauf 22) und einen Messwertverlauf 24, nämlich einen Verlauf des Messsignals S des Gassensors 10, wobei in dem Messwertverlauf 24 einzelne Messwerte 26 des Gassensors 10 hervorgehoben sind. Anhand des Volumenstromverlaufs 22 ist erkennbar, dass im Rahmen des Kalibrierungsverfahrens 20 der Volumenstrom Q von einem Anfangswert schrittweise bis zu einem Zielwert reduziert wird. Die Reduktion erfolgt mit einer vorgegebenen oder vorgebbaren Schrittweite. Ausgehend von dem Anfangswert und nach jeder Reduktion wird der Volumenstrom Q für eine vorgegebene oder vorgebbare Zeitspanne konstant gehalten. Während dieser Zeit eines konstanten Volumenstroms Q (Volumenstromplateau) erfolgt die Aufnahme (Messung) eines Messwerts 26 (Kalibrierungs-Gasmesswert) des Gassensors 10. Die einzelnen Messwerte 26 sind in der Darstellung in Figur 3 auf dem Messwertverlauf 24 graphisch hervorgehoben.

In dem Messwertverlauf 24 ist deutlich eine Abhängigkeit der Messwerte 26 (Kalibrierungs-Gasmesswerte) vom jeweiligen Volumenstrom Q (Kalibrierungs-Volumenstromwert) erkennbar. Im Rahmen der Kalibrierung ergibt sich auf Basis eines jeweiligen Volumenstroms Q und eines zugehörigen Messwerts 26 ein volumenstromabhängiger Korrekturfaktor 28. Der Entwicklung des Korrekturfaktors 28 ist in der Darstellung in Figur 4 als Korrekturfaktorverlauf 30 über dem Volumenstrom Q dargestellt. Aus der Gesamtheit der Korrekturfaktoren 28 ergibt sich - zum Beispiel durch eine einfache Interpolation oder eine Polynominterpolation auf Basis der Korrekturfaktoren 28 - eine Kalibrierfunktion 32.

Die Kalibrierfunktion 32 oder die zugrunde liegenden Korrekturfaktoren 28 werden bei der Kalibrierung eines vom Gassensor 10 beim späteren Messbetrieb gelieferten Messwerts 26 (Betriebs-Gasmesswert) verwendet. Die Kalibrierfunktion 32 oder die zugrunde liegenden Korrekturfaktoren 28 fungiert bzw. fungieren als Kalibrierungsbasis. Dabei wird abhängig von einem beim Messbetrieb jeweils herrschenden Volumenstrom Q (Betriebs-Volumenstromwert) ein zu diesem Volumenstrom Q am besten passender Korrekturfaktor 28 oder ein zu diesem Volumenstrom Q gehöriger Wert der Kalibrierfunktion 32 ermittelt und dieser zum Erhalt eines kalibrierten Messwerts mit dem originären Messwert 26 (Betriebs-Gasmesswert) verknüpft, zum Beispiel multiplikativ verknüpft: kalibrierter Messwert = originärer Messwert 26 / Korrekturfaktor 28.

Die Darstellung in Figur 5 zeigt schematisch vereinfacht den Ablauf eines Kalibrierungsverfahrens 20 der hier vorgeschlagenen Art. Nach Abschluss einer finalen Installation eines Gassensors 10 und einer Pumpe 12 in einem Schlauchsystem 14 - zum Beispiel wie in Figur 1 gezeigt - wird das Kalibrierungsverfahren 20 mit einem ersten Schritt 34 gestartet.

In diesem ersten Schritt 34 werden die Pumpe 12 gestartet und ein Prüfgas 16 bereitgestellt. Die Prüfgaskonzentration wird während des Kalibrierungsverfahrens 20 konstant gehalten. Die Pumpe 12 wird - zum Beispiel durch Auswahl einer entsprechenden Drehzahl - mit einem vorgegebenen oder vorgebbaren Startwert für einen hohen Volumenstrom Qs gestartet. Durch das Aktivieren der Pumpe 12 wird das Prüfgas 16 in das Schlauchsystem 14 gesaugt und zum Gassensor 10 gefördert. In einem zweiten Schritt 36 erfolgt eine Messung in Form einer Erfassung eines Messwerts 26 (Kalibrierungs-Gasmesswert) mittels des Gassensors 10. Dabei kann bis zur Aufnahme oder Verarbeitung eines Messwerts 26 zum Beispiel der Ablauf einer vorgegebenen oder vorgebbaren Totzeit abgewartet werden, um anfängliche Messwertschwankungen während einer "Einschwingzeit" zu ignorieren. Zusätzlich oder alternativ kann bei der Aufnahme eines Messwerts 26 berücksichtigt werden, ob der Messwert 26 in einem Erwartungsrahmen liegt und/oder ob die Schwankungen des Messwerts 26 während der Messung unterhalb eines vorgegebenen oder vorgebbaren Schwellwerts ΔQ verbleiben. Ein aufgenommener Messwert 26 (Kalibrierungs-Gasmesswert) oder ein aus einer Mehrzahl aufgenommener Messwerte 26 (Kalibrierungs-Gasmesswerte) gebildeter Mittelwert wird zusammen mit einem den jeweiligen Volumenstrom Q kodierenden (oder ausdrückenden) Kalibrierungs-Volumenstromwert als volumenstromabhängiger Messwert (volumenstromabhängiger Kalibrierungs-Gasmesswert) M_{Q} gespeichert. Danach wird in einem dritten Schritt 38 geprüft, ob der Volumenstrom Q noch größer als ein vorgegebener oder vorgebbarer Endwert Q_{E} für den Volumenstrom ist. Wenn dies der Fall ist (Zweig "+"), wird der Volumenstrom Q in einem vierten Schritt 40 entsprechend einer vorgegebenen oder vorgebbaren Schrittweite Qᵢ reduziert und danach zum zweiten Schritt 36 verzweigt, wo die Messung mit dem Ergebnis der Ermittlung eines weiteren volumenstromabhängigen Messwerts M_{Q} fortgesetzt wird. Wenn im dritten Schritt 38 festgestellt wird, dass der Volumenstrom Q bereits den Endwert Q_{E} erreicht hat, ist das Verfahren beendet und es wird zu einem das Kalibrierungsverfahren 20 abschließenden fünften Schritt 42 verzweigt (Zweig "-"). Hier wird zum Beispiel auf Basis der Gesamtheit der im Rahmen des Kalibrierungsverfahrens 20 aufgenommenen Kalibrierungs-Volumenstromwerte sowie der volumenstromabhängigen Messwerte (volumenstromabhängige Kalibrierungs-Gasmesswerte) M_{Q} als Kalibrierungsbasis die Kalibrierfunktion 32 (Fig. 4) gebildet.

Danach kann der normale Messbetrieb beginnen. Beim Messbetrieb wird ein originärer Messwert (Betriebs-Gasmesswert) 26 vom Gassensor 10 in Abhängigkeit von einem jeweils wirksamen Volumenstrom Q und einem diesen kodierenden (oder ausdrückenden) Betriebs-Volumenstromwert anhand der Kalibrierungsbasis kalibriert, also zum Beispiel mit dem Wert der Kalibrierfunktion 32 an der Stelle des jeweiligen Volumenstroms Q (Betriebs-Volumenstromwert) kalibriert.

Die Ausführung des Kalibrierungsverfahrens 20 erfolgt unter Kontrolle einer Steuerungseinheit 44. Diese umfasst in grundsätzlich an sich bekannter Art und Weise zum Beispiel eine Verarbeitungseinheit in Form von oder nach Art eines Mikroprozessors sowie einen Speicher 46, in den ein Steuerungsprogramm 48 geladen wird oder ist, das beim Betrieb der Steuerungseinheit 44 durch dessen Verarbeitungseinheit ausgeführt wird.

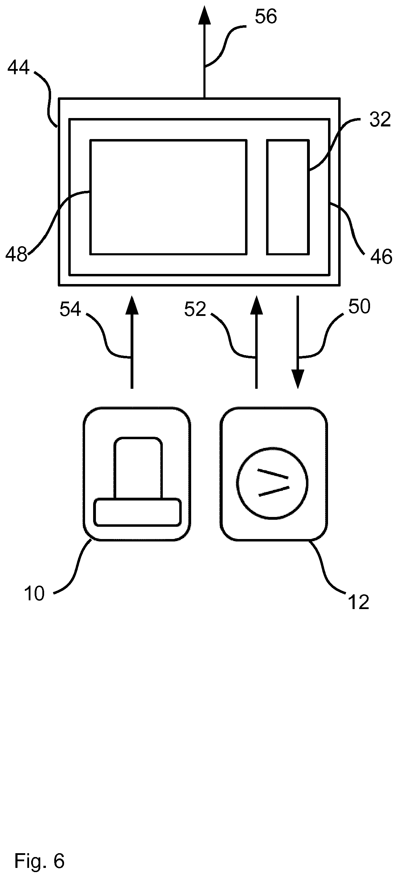

Eine solche Steuerungseinheit 44 ist schematisch vereinfacht in der Darstellung in Figur 6 gezeigt und ist optional der Pumpe 12 zugeordnet, zum Beispiel derart, dass die Pumpe 12 die Steuerungseinheit 44 umfasst und die Steuerungseinheit 44 weitere, den Betrieb der Pumpe 12 betreffende Steuerungs- und/oder Überwachungsfunktionen erfüllt. Je nachdem, welcher Einheit - Pumpe 12 oder Gassensor 10 - die Steuerungseinheit 44 zugeordnet ist, erfolgt eine Übertragung von Daten von und/oder zu der jeweiligen Einheit zum Beispiel intern und mit der jeweils anderen Einheit ist die Steuerungseinheit 44 in einer eine solche Datenübertragung ermöglichenden und grundsätzlich an sich bekannten Art und Weise kommunikativ verbunden.

Bei der Kalibrierung eines Gassensors 10 bestimmt die Steuerungseinheit 44 entsprechend dem Steuerungsprogramm 48 den von der Pumpe 12 im Betrieb erzeugten Volumenstrom Q. Dafür wird von der Steuerungseinheit 44 ein Steuersignal 50 an die Pumpe 12 ausgegeben. Bei einer Regelung des Volumenstroms Q mittels der Steuerungseinheit 44 empfängt diese für die Regelung von der Pumpe 12 ein Istwertsignal 52, welches einen Momentanwert des Volumenstroms Q kodiert. Das Steuersignal 50 oder das Istwertsignal 52 kann als Volumenstromwert oder als Basis für einen Volumenstromwert verwendet werden, nämlich während des Kalibrierungsverfahrens als Kalibrierungs-Volumenstromwert oder als Basis für den Kalibrierungs-Volumenstromwert. Zusätzlich oder alternativ kommt in Betracht, mittels einer entsprechenden Sensorik den jeweils wirksamen Volumenstrom Q zu erfassen und einen entsprechenden Messwert als Kalibrierungs-Volumenstromwert zu verwenden. Vom Gassensor 10 empfängt die Steuerungseinheit 44 ein einen jeweils aktuellen originären Messwert 26 (Kalibrierungs-Gasmesswert) repräsentierendes Messwertsignal 54.

Durch Vorgabe jeweils eines Steuersignals 50 entsprechend dem im Rahmen des Kalibrierungsverfahrens 20 geforderten Volumenstrom Q und Auswertung des Messwertsignals 54 vom Gassensor 10 wird unter Kontrolle der Steuerungseinheit 44 das Kalibrierungsverfahren 20 ausgeführt und die dabei ermittelten volumenstromabhängigen Messwerte M_{Q} (volumenstromabhängige Kalibrierungs-Gasmesswerte) werden zusammen mit dem jeweiligen Volumenstrom Q (Kalibrierungs-Volumenstromwert) in dem Speicher 46 der Steuerungseinheit 44 abgelegt. Die volumenstromabhängigen Messwerte M_{Q} und der jeweils zugehörige Volumenstrom Q bilden die Kalibrierungsbasis. Optional wird daraus die Kalibrierfunktion 32 ermittelt und ebenfalls im Speicher 46 abgelegt.

Beim Messbetrieb wird mittels der Pumpe 12 anstelle eines Prüfgases 16 (Fig. 1) ein zu messendes Gas angesaugt. Das Messwertsignal 54 des Gassensors 10 repräsentiert dann eine gemessene Gaskonzentration dieses Gases. Beim Messbetrieb verarbeitet die Steuerungseinheit 44 zum einen das Messwertsignal 54 als aktuellen Messwert (Betriebs-Gasmesswert) und zum anderen zum Beispiel das Istwertsignal 52 oder - bei einer ungeregelten Pumpe 12 - das Steuersignal 50 als aktuellen Volumenstrom Q (Betriebs-Volumenstromwert). Auch hier, also während des Messbetriebs, kann das Steuersignal 50 oder das Istwertsignal 52 als Volumenstromwert oder als Basis für einen Volumenstromwert verwendet werden, nämlich während des Messbetriebs als Betriebs-Volumenstromwert oder als Basis für den Betriebs-Volumenstromwert. Ebenso kommt auch hier in Betracht, mittels einer entsprechenden Sensorik den jeweils wirksamen Volumenstrom Q zu erfassen und einen entsprechenden Messwert als Betriebs-Volumenstromwert zu verwenden.

Mit dem aktuellen Volumenstrom Q (Betriebs-Volumenstromwert) und der Kalibrierungsbasis, insbesondere der Kalibrierfunktion 32, ergibt sich ein volumenstromabhängiger Korrekturfaktor 28 und mit diesem wird der Messwert gewichtet. Der gewichtete (kalibrierte) Messwert wird als Gasmesswert 56 ausgegeben.

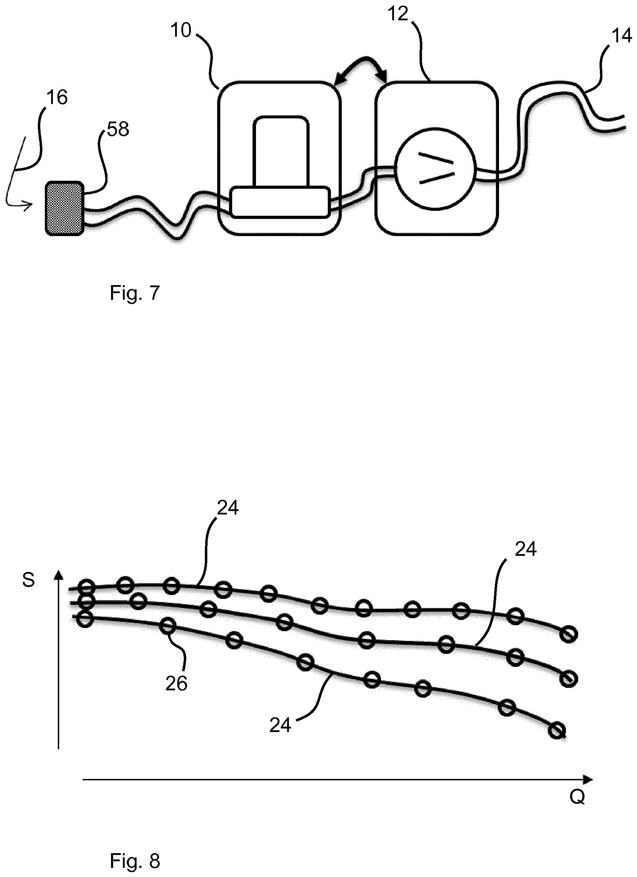

Bei einer besonderen Variante des hier vorgeschlagenen Kalibrierungsverfahrens wird zusätzlich zu einem jeweils wirksamen Volumenstrom Q (Kalibrierungs-Volumenstromwert) die Wirkung eines Filters 58 (Fig. 7) erfasst. Dann ergeben sich filterabhängige oder allgemein strömungswiderstandsabhängige Messwertverläufe 24 und jeweils zugehörige Messwerte 26 (Kalibrierungs-Gasmesswerte). Die Darstellung in Figur 8 zeigt insoweit exemplarisch drei Messwertverläufe 24, die jeweils für ein Schlauchsystem 14 mit ein und demselben eingangsseitigen Filter 58 aufgenommen wurden, wobei der oberste Messwertverlauf 24 die Verhältnisse bei einem frischen Filter 58 unmittelbar nach dessen Installation repräsentiert. Der untere Messwertverlauf 24 repräsentiert die Verhältnisse bei einem belegten Filter 58 und der Messwertverlauf 24 zwischen dem oberen Messwertverlauf 24 und dem unteren Messwertverlauf 24 repräsentiert die Verhältnisse bei einem in etwa zur Hälfte belegten Filter 58.

Die Aufnahme der Messwerte 26 (Kalibrierungs-Gasmesswerte) erfolgt wie zuvor anhand von Figur 3 und Figur 5 beschrieben. Der einzige Unterschied besteht darin, dass die Aufnahme der Messwerte 26 ggf. mehrfach erfolgt, nämlich für unterschiedliche Verhältnisse, also zum Beispiel für unterschiedliche wirksame Strömungswiderstände jeweils einmal. Für jeden resultierenden Messwertverlauf 24 ergibt sich eine Kalibrierungsbasis, zum Beispiel ein Korrekturfaktorverlauf 30 und/oder eine Kalibrierfunktion 32 wie in Figur 4 gezeigt, nämlich eine strömungswiderstandsabhängige Kalibrierungsbasis, insbesondere ein strömungswiderstandsabhängiger Korrekturfaktorverlauf 30 und/oder eine strömungswiderstandsabhängige Kalibrierfunktion 32. Diese wird bzw. werden zum Beispiel mit einer Kategorie versehen und unter der jeweiligen Kategorie abgespeichert, zum Beispiel "frischer Filter", "teilweise belegter Filter", "belegter Filter". Eine Auswahl einer Kalibrierungsbasis, eines Korrekturfaktors 28, eines Korrekturfaktorverlaufs 30 oder einer Kalibrierfunktion 32 erfolgt auf Basis von Messwerten, die mittels einer der Pumpe 12 zugeordneten Sensorik aufgenommen werden. Bei den Messwerten handelt es sich zusätzlich zu dem bereits in der bisherigen Erläuterung erwähnten Volumenstrommesswert um einen Unterdruckmesswert. Aus diesen Messwerten ergibt sich in grundsätzlich an sich bekannter Art und Weise ein jeweils wirksamer Strömungswiderstand und daraus kann zum Beispiel auf einen jeweiligen Zustand eines Filters 58 geschlossen werden. Anhand der Messwerte (Volumenstrommesswert, Unterdruckmesswert) ergibt sich also eine Kategorisierung für den aktuellen Belegungszustand des Filters 58 und auf dieser Basis kann die Auswahl einer Kalibrierungsbasis, eines Korrekturfaktors 28, eines Korrekturfaktorverlaufs 30 oder einer Kalibrierfunktion 32 erfolgen. Selbstverständlich können mehr oder weniger als drei unterschiedliche Kategorien zur Anwendung kommen.

Optional kann für einzelne oder mehrere Umgebungsparameter jeweils eine weitere Kalibrierungsbasis oder Kennlinie entsprechend dem in Figur 3 gezeigten Messwertverlauf 24 aufgenommen werden. Anstelle der dort (Fig. 3) erfolgenden Variation des Volumenstroms Q werden dann zum Beispiel unterschiedliche Temperaturen, unterschiedliche Luftfeuchtigkeiten usw. betrachtet. Eine Auswahl einer insoweit aufgenommenen Kalibrierungsbasis und eine Ermittlung eines sich daraus ergebenden Korrekturfaktors erfolgt anhand eines mittels eines Temperatur- und/oder Luftfeuchtesensors usw. ermittelten Messwerts. Bei einer Unabhängigkeit der den originären Messwert 26 des Gassensors 10 beeinflussenden Faktoren können die jeweils resultierenden Korrekturfaktoren multiplikativ angewandt werden. Zur Erfassung einer Kreuzabhängigkeit - zum Beispiel Volumenstrom und Temperatur - erfolgt im Rahmen des Kalibrierungsverfahrens 20 eine Variation des Volumenstroms Q und eine darin jeweils eingebettete Variation der Temperatur (oder umgekehrt), so dass sich als Kalibrierungsbasis zum Beispiel keine einzelne Kennlinie, wie bei dem in Figur 3 gezeigten Messwertverlauf 24, sondern ein Kennlinienfeld (nicht gezeigt) ergibt. Beim Messbetrieb wird dann entsprechend dem jeweils wirksamen Volumenstrom Q und der ermittelten Umgebungstemperatur ein Punkt in dem Kennlinienfeld ermittelt und daraus ein Korrekturfaktor abgeleitet.

Einzelne besonders im Vordergrund stehende Aspekte der hier vorgelegten Beschreibung lassen sich damit kurz wie folgt zusammenfassen: Angegeben werden ein Verfahren zum eine Kalibrierung umfassenden Betrieb eines Gassensors 10, wobei mittels einer mit dem Gassensor 10 gekoppelten Pumpe 12 ein Prüfgas 16 eingesaugt und zum Gassensor 10 gefördert wird, wobei mittels der Pumpe 12 unterschiedliche Volumenströme Q erzeugt werden und mittels des Gassensors 10 jeweils volumenstromabhängige Messwerte 26 (Kalibrierungs-Gasmesswerte) aufgenommen werden und wobei die volumenstromabhängigen Messwerte 26 zusammen mit dem jeweiligen Volumenstrom Q (Kalibrierungs-Volumenstromwert) aufgezeichnet werden, sowie ein als Steuerungsprogramm 48 fungierendes Computerprogramm mit einer Implementation des Verfahrens.

### BEZUGSZEICHENLISTE

- 10: Gassensor
- 12: Pumpe
- 14: Schlauchsystem
- 16: Prüfgas
- 18: (frei)
- 20: Kalibrierungsverfahren
- 22: Volumenstromverlauf
- 24: Messwertverlauf
- 26: Messwert
- 28: Korrekturfaktor
- 30: Korrekturfaktorverlauf
- 32: Kalibrierfunktion
- 34: erster Schritt (des Kalibrierungsverfahrens)
- 36: zweiter Schritt
- 38: dritter Schritt
- 40: vierter Schritt
- 42: fünfter Schritt
- 44: Steuerungseinheit
- 46: Speicher
- 48: Steuerungsprogramm
- 50: Steuersignal
- 52: Istwertsignal
- 54: Messwertsignal
- 56: Gasmesswert
- 58: Filter

## Patentansprüche

1. Verfahren zum Betrieb eines Gassensors (10),
wobei mittels einer mit dem Gassensor (10) gekoppelten Pumpe (12) ein Prüfgas (16) eingesaugt und zum Gassensor (10) gefördert wird,
wobei mittels der Pumpe (12) unterschiedliche Volumenströme erzeugt werden und mittels des Gassensors (10) jeweils volumenstromabhängige Messwerte (26) aufgenommen werden,
wobei die volumenstromabhängigen Messwerte (26) zusammen mit dem jeweiligen Volumenstrom aufgezeichnet werden,
wobei auf Basis der volumenstromabhängigen Messwerte (26) und dem jeweils zugehörigen Volumenstrom als Kalibrierungsbasis Korrekturfaktoren (28) oder eine Kalibrierfunktion (32) ermittelt werden,
wobei bei einem Messbetrieb des Gassensors (10)
einerseits ein von dem Gassensor (10) gelieferter Messwert (26) und ein wirksamer Volumenstrom erfasst werden und
andererseits der erfasste Messwert (26) anhand des erfassten wirksamen Volumenstroms sowie anhand der im Rahmen des Kalibrierungsverfahrens ermittelten volumenstromabhängigen Messwerte (26) korrigiert wird.

2. Verfahren nach Anspruch 1,
wobei der erfasste Messwert (26) korrigiert wird, indem die Kalibrierfunktion (32) oder die Korrekturfaktoren (28) zur Kalibrierung des erfassten Messwerts (26) verwendet werden, indem abhängig von dem erfassten wirksamen Volumenstrom ein zu diesem am besten passender Korrekturfaktor (28) oder ein zu diesem gehöriger Wert der Kalibrierfunktion (32) ermittelt und der Messwert (26) damit kalibriert wird.

3. Verfahren nach Anspruch 1 oder 2,
wobei zur Erzeugung unterschiedlicher Volumenströme mittels der Pumpe (12) anfänglich ein Volumenstrom entsprechend einem vorgegebenen Startwert erzeugt wird und wobei ausgehend von dem Startwert der Volumenstrom erhöht oder verringert wird, bis ein vorgegebener Zielwert erreicht ist.

4. Verfahren nach Anspruch 3,
wobei der Volumenstrom ausgehend vom dem Startwert mit einer vorgegebenen oder vorgebbaren Schrittweite schrittweise erhöht oder verringert wird, bis der Zielwert erreicht ist.

5. Verfahren nach einem der Ansprüche 3 oder 4, wobei der Startwert für den Volumenstrom größer als der Zielwert ist und wobei der Volumenstrom ausgehend von dem Startwert verringert wird, bis der Zielwert erreicht ist.

6. Verfahren nach einem der vorangehenden Ansprüche,
wobei die für die Kalibrierungsbasis aufgenommenen volumenstromabhängigen Messwerte (26) für unterschiedliche Strömungswiderstände aufgenommen werden.

7. Verfahren nach einem der vorangehenden Ansprüche,
wobei anhand des erfassten Volumenstroms eine Kalibrierungsbasis ausgewählt wird und wobei der erfasste Messwert (26) anhand der ausgewählten Kalibrierungsbasis korrigiert wird.

8. Verfahren nach einem der vorangehenden Ansprüche,
wobei die in die oder jede Kalibrierungsbasis eingehenden volumenstromabhängigen Messwerte (26) aufgenommen werden, wenn sich der Gassensor (10) in einer für den späteren Betrieb des Gassensors (10) vorgesehenen Einbausituation befindet.

9. System mit einem Gassensor (10) und einer mit dem Gassensor (10) fluidisch gekoppelten Pumpe (12) mit einer Steuerungseinheit (44) und einem mittels der Steuerungseinheit (44) ausführbaren Steuerungsprogramm (48) mit einer Implementation des Verfahrens nach einem der Ansprüche 1 bis 8.

10. Steuerungsprogramm (48) in Form eines Computerprogramms mit Programmcodemitteln, welche bewirken, dass das System nach Anspruch 9 die Verfahrensschritte nach einem der Ansprüche 1 bis 8 ausführt.

## Claims

1. Method for operating a gas sensor (10),
wherein a test gas (16) is drawn in and fed to the gas sensor (10) by means of a pump (12) coupled to the gas sensor (10),
wherein different volume flows are produced by means of the pump (12) and volume-flow-dependent measured values (26) are in each case recorded by means of the gas sensor (10),
wherein the volume-flow-dependent measured values (26) are recorded together with the respective volume flow,
wherein correction factors (28) or a calibrating function (32) are determined on the basis of the volume-flow-dependent measured values (26) and the respectively associated volume flow as a basis for calibration,
wherein, in a measuring mode of the gas sensor (10), on the one hand a measured value (26) provided by the gas sensor (10) and an effective volume flow are detected and
on the other hand the detected measured value (26) is corrected on the basis of the detected effective volume flow and on the basis of the volume-flow-dependent measured values (26) determined in the course of the calibration process.

2. Method according to Claim 1,
wherein the detected measured value (26) is corrected by the calibrating function (32) or the correction factors (28) being used for calibrating the detected measured value (26), by determining - dependent on the detected effective volume flow - a correction factor (28) best suited for it or a value of the calibrating function (32) associated with it, and the measured value (26) being calibrated with it.

3. Method according to Claim 1 or 2,
wherein, for producing different volume flows by means of the pump (12), initially a volume flow is produced in accordance with a preselected starting value and wherein, starting from the starting value, the volume flow is increased or reduced until a preselected target value is reached.

4. Method according to Claim 3,
wherein, starting from the starting value, the volume flow is increased or reduced in steps of a preselected or preselectable increment until the target value is reached.

5. Method according to either of Claims 3 and 4, wherein the starting value for the volume flow is greater than the target value and wherein, starting from the starting value, the volume flow is reduced until the target value is reached.

6. Method according to one of the preceding claims,
wherein the volume-flow-dependent measured values (26) recorded as a basis for calibration are recorded for different flow resistances.

7. Method according to one of the preceding claims,
wherein a basis for calibration is selected on the basis of the detected volume flow and wherein the detected measured value (26) is corrected on the basis of the selected basis for calibration.

8. Method according to one of the preceding claims,
wherein the volume-flow-dependent measured values (26) included in the or each basis for calibration are recorded when the gas sensor (10) is in an installation situation intended for subsequent operation of the gas sensor (10).

9. System with a gas sensor (10) and a pump (12) fluidically coupled to the gas sensor (10) with a control unit (44) and a control program (48) that can be executed by means of the control unit (44) with implementation of the method according to one of Claims 1 to 8.

10. Control program (48) in the form of a computer program with program coding means, which have the effect that the system according to Claim 9 performs the method steps according to one of Claims 1 to 8.

## Revendications

1. Procédé de fonctionnement d'un capteur de gaz (10), un gaz de test (16) étant aspiré au moyen d'une pompe (12) accouplée au capteur de gaz (10) et transporté vers le capteur de gaz (10),
différents débits volumiques étant générés au moyen de la pompe (12) et des valeurs de mesure (26) dépendant des débits volumiques étant acquises au moyen du capteur de gaz (10),
les valeurs de mesure (26), dépendant des débits volumiques, étant acquises conjointement avec le débit volumique respectif,
des facteurs de correction (28) ou une fonction d'étalonnage (32) étant déterminés comme base d'étalonnage sur la base des valeurs de mesure (26) dépendant des débits volumiques et du débit volumique respectivement associé,
lors d'une opération de mesure du capteur de gaz (10) une valeur de mesure (26), fournie par le capteur de gaz (10), et un débit volumique effectif étant d'une part détectés et
la valeur de mesure détectée (26) étant d'autre part corrigée sur la base du débit volumique effectif détecté et sur la base des valeurs de mesure (26) dépendant des débits volumiques et déterminées dans le cadre du procédé d'étalonnage.

2. Procédé selon la revendication 1,
la valeur de mesure détectée (26) étant corrigée à l'aide de la fonction d'étalonnage (32) ou des facteurs de correction (28) afin d'étalonner la valeur de mesure détectée (26) par détermination, en fonction du débit volumique effectif détecté, d'un facteur de correction (28) correspondant le mieux à celui-ci, ou d'une valeur de la fonction d'étalonnage (32) associée à celui-ci et ainsi par étalonnage de la valeur de mesure (26).

3. Procédé selon la revendication 1 ou 2,
un débit volumique correspondant à une valeur de départ spécifiée étant initialement généré afin de générer différents débits volumiques au moyen de la pompe (12), et
le débit volumique étant augmenté ou diminué à partir de la valeur de départ jusqu'à ce qu'une valeur cible spécifiée soit atteinte.

4. Procédé selon la revendication 3,
le débit volumique étant progressivement augmenté ou diminué à partir de la valeur de départ avec un incrément spécifié ou spécifiable jusqu'à ce que la valeur cible soit atteinte.

5. Procédé selon l'une des revendications 3 ou 4,
la valeur de départ du débit volumique étant supérieure à la valeur cible et
le débit volumique étant réduit à partir de la valeur de départ jusqu'à ce que la valeur cible soit atteinte.

6. Procédé selon l'une des revendications précédentes, les valeurs de mesure (26), dépendant des débits volumiques et acquises pour la base d'étalonnage, étant acquises pour différentes résistances à l'écoulement.

7. Procédé selon l'une des revendications précédentes, une base d'étalonnage étant sélectionnée sur la base du débit volumique détecté et
la valeur de mesure détectée (26) étant corrigée à l'aide de la base d'étalonnage sélectionnée.

8. Procédé selon l'une des revendications précédentes, les valeurs de mesure (26), dépendant des débits volumiques et entrant dans la ou chaque base d'étalonnage étant acquises lorsque le capteur de gaz (10) se trouve dans une situation d'installation prévue pour un fonctionnement ultérieur du capteur de gaz (10).

9. Système comprenant un capteur de gaz (10) et une pompe (12) accouplée fluidiquement au capteur de gaz (10) et comprenant une unité de commande (44) et un logiciel de commande (48) exécutable au moyen de l'unité de commande (44) avec mise en œuvre du procédé selon l'une des revendications 1 à 8.

10. Programme de commande (48) se présentant sous la forme d'un logiciel comprenant des moyens de code de programme qui amènent le système selon la revendication 9 à exécuter les étapes de procédé selon l'une des revendications 1 à 8.
